# EUROPEAN PATENT APPLICATION

(11) **EP 4 501 944 A1**
(43) Date of publication of application: **05.02.2025**
(21) Application number: 22933971.8
(22) Date of filing: 28.03.2022
(51) Int. Cl.: C07K 7/06, A61K 38/08, A61P 17/00, A61Q 19/00

(54) **POLYPEPTIDE AND COSMETIC COMPOSITION OR PHARMACEUTICAL COMPOSITION AND USE THEREOF**

(71) Applicant: Shenzhen Winkey Technology Co., Ltd, Shenzhen, Guangdong 518000 (CN)
(72) Inventor: DING, Wenfeng, Shenzhen, Guangdong 518000 (CN); XIAO, Yu, Guangdong 518000 (CN); ZHAO, Wenhao, Shenzhen, Guangdong 518000 (CN); SUN, Xinlin, Shenzhen, Guangdong 518000 (CN); CHEN, Xue, Shenzhen, Guangdong 518000 (CN); GUAN, Fuyi, Shenzhen, Guangdong 518000 (CN)
(74) Representative: Bayramoglu et al.
(86) International application number: PCT/CN2022/083491
(87) International publication number: WO 2023/184114

(57) **Abstract**

The present application provides a polypeptide and a cosmetic composition or pharmaceutical composition and use thereof. The polypeptide has the following general formula: R₁-X₁-X₂-X₃-X₄-X₅-X₆-X₇-X₈-R₂. The present application relates to a peptide, stereoisomers thereof, a mixture of the stereoisomers, or a cosmetically acceptable salt thereof, or a pharmaceutically acceptable salt thereof, or a cosmetic composition or a pharmaceutical composition thereof, and a use thereof in the preparation of the cosmetic composition or pharmaceutical composition for improving skin aging or photoaging, and treating or caring for a condition, a disorder or a disease caused by muscle contraction.

## Description

### TECHNICAL FIELD

The present application relates to the technical field of medicine and cosmetics, and particularly relates to a polypeptide and cosmetic composition or pharmaceutical composition and use thereof.

### BACKGROUND TECHNOLOGY

The most obvious sign of aging of human skin is the formation of facial wrinkles. With the passage of time, influenced by various factors, such as an increase of age, external environment, and genetic factors, the neural activity of human body becomes excessive, and neurotransmitters that stimulate muscle fibers are released uncontrollably and excessively, which drags the muscle tension of the skin inward and deepens the first-level line on the skin surface to form wrinkles. At the cellular level, fibroblasts that synthesize extracellular matrix and collagen on the tension line can exhibit specific contractile properties related to striated muscles under the action of muscle contraction. The junction between nerves and striated muscles forms the neuromuscular plate, upstream of which is the pathway for motor neuron afferent nerves. It is known that facial skin muscles are under the control of motor nerve afference, and acetylcholine is released from synaptic vesicles and acts on acetylcholine receptors in the postsynaptic membrane to cause muscle contraction, which leads to the formation of wrinkles.

The vesicles need to be fused with the presynaptic membrane first in order to complete the exocytosis process and release acetylcholine. The membrane fusion is mediated by the N-ethylmaleimide sensitive factor attachment protein receptor (SNARE) complexes. The SNARE complexes have a four-helix bundle structure composed of synaptic vesicle associated membrane protein (VAMP2) on the vesicle membrane, synaptic associated protein (SNAP25) on the presynaptic membrane, and synaptic fusion protein (Syntaxin). SNARE participates in neuronal exocytosis and muscle contraction activity by undergoing conformational changes during aggregation and releasing energy, and promoting the fusion of the vesicle membrane and presynaptic membrane and the release of neurotransmitters. Synaptophysin is a synaptic vesicle transmembrane glycoprotein present in presynaptic vesicles of neurons and neurosecretory granules of neuroendocrine cells, and plays an important role in the release of neurotransmitters. Synaptophysin may interact with VAMP2 and be separated from VAMP2 before VAMP2 forms SNARE complexes with SNAP25 and Syntaxin, thereby regulating the formation of the SNARE complexes and leading to reduced release of neurotransmitters under high-frequency stimulation. Abnormal regulation of synaptophysin will lead to abnormal neuronal exocytosis and muscle contraction disorders.

At present, several methods for reducing and/or eliminating expression wrinkles have been discovered by targeting the SNARE protein complexes. For example, local injection of botulinum toxin A is used for wrinkle removal, facial slimming, and elimination of wrinkles between eyebrows and periorbital wrinkles, but there is a risk of triggering an immune response and losing the therapeutic effect; peptides derived from SNAP-25 amino and carboxyl domains disclosed in patents WO 0064932, EP 1180524B1 and EP 2123673B1; and peptides derived from Munc18 disclosed in WO 2019166347. However, compounds that can effectively inhibit muscle contraction in a manner similar to botulinum toxin but without risk are not yet available at present. Therefore, there is still a need to search for compounds with other mechanisms of action so as to inhibit neuronal exocytosis, interfere with the release of synaptic neurotransmitters, and meet the growing demand for skin treatment or care.

### CONTENT OF THE INVENTION

The inventors of the present application have studied and found that polypeptide fragments derived from Synaptophysin may interfere with the interaction between Synaptophysin and VAMP2 and thus inhibit the formation of SNARE complexes and the release of neurotransmitters (preferably acetylcholine), thereby inhibiting muscle contraction. Therefore, they can be used for the treatment or care of conditions, disorders, or diseases caused by muscle contraction, as well as for the treatment or care of the skin.

In this regard, the present application provides a peptide represented by Formula (I), or a stereoisomer thereof, or a mixture of the stereoisomers thereof, or a cosmetically acceptable salt thereof, or a pharmaceutically acceptable salt thereof,

R₁-X₁-X₂-X₃-X₄-X₅-X₆-X₇-X₈-R₂ (I)

in Formula (I),
X₁ is selected from -Val-, -Ser-, -Trp-, -Met-, -Leu-, -Glu-, -Ala-, -Pro-, -Tyr- or -Thr-;
X₂ is selected from -Lys-, -Arg-, -His-, -Met-, -Ala-, -Leu-, -Asn-, -Trp-, -Thr-, -Pro-, -Gly-, - Phe-, -Asp-, -Ser- or -Cys-;
X₃ is selected from -Val-, -Gly-, -Leu-, -Pro-, -Met-, -Ala-, -Gln-, -Asp-, -Phe-, -Ser-, -Cys- or - Lys-;
X₄ is selected from -Gly-, -Leu-, -Pro-, -Ala-, -Phe-, -Val-, -Trp-, -Asn- or -Glu-;
X₅ is selected from -Phe-, -Trp-, -Tyr- or a bond;
X₆ is selected from -Gln-, -Leu-, -Ala-, -Val-, -Phe-, -Arg-, -Tyr-, -Gly- or -Asn-;
X₇ is selected from -Lys-, -Arg-, -His- or a bond;
X₈ is selected from -Trp-, -Ala-, -Asp-, -Lys-, -Gly-, -Tyr-, -Met- or -Arg-;
R₁ is selected from H or R₃-CO-, wherein R₃ is selected from substituted or unsubstituted alkyl, substituted or unsubstituted alkenyl;
R₂ is selected from -NR₄R₅ or -OR₄, wherein each R₄ and R₅ are independently selected from H, substituted or unsubstituted alkyl, substituted or unsubstituted alkenyl;
the alkyl refers to a saturated aliphatic linear or branched chain alkyl having 1-24 carbon atoms (optionally having 1-16 carbon atoms; optionally having 1-14 carbon atoms; optionally having 1-12 carbon atoms; or optionally having 1, 2, 3, 4, 5, or 6 carbon atoms); optionally selected from methyl, ethyl, isopropyl, isobutyl, *tert*-butyl, pentyl, hexyl, heptyl, octyl, decyl, dodecyl, tetradecyl, hexadecyl, octadecyl, 2-ethylhexyl, 2-methylbutyl, or 5-methylhexyl;
the alkenyl refers to a linear or branched chain alkenyl having 2-24 carbon atoms (optionally having 2-16 carbon atoms; optionally having 2-14 carbon atoms; optionally having 2-12 carbon atoms; or optionally having 2, 3, 4, 5, or 6 carbon atoms); the alkenyl has one or more carbon-carbon double bonds, optionally has 1, 2, or 3 conjugated or non-conjugated carbon-carbon double bonds; the alkenyl is bonded to the rest portions of a molecule through a single bond; and is optionally selected from vinyl, oleyl, or linoleyl;
optionally, the substituent in the "substituted alkyl" or "substituted alkenyl" is selected from C₁-C₄ alkyl; hydroxyl; C₁-C₄ alkoxy; amino; C₁-C₄ aminoalkyl; C₁-C₄ carbonyloxy; C₁-C₄ oxycarbonyl; halogen (such as fluorine, chlorine, bromine, and iodine); cyano; nitro; azide; C₁-C₄ alkylsulfonyl; thiol; C₁-C₄ alkylthio; C₆-C₃₀ aryloxy such as phenoxy; -NR_{b}(C=NR_{b}) NR_{b}R_{c}, wherein R_{b} and R_{c} are independently selected from H, C₁-C₄ alkyl, C₂-C₄ alkenyl, C₂-C₄ alkynyl, C₃-C₁₀ cycloalkyl, C₆-C₁₈ aryl, C₇-C₁₇ aralkyl, a heterocyclic group having 3 to 10 members, or an amino protecting group.

Further, R₁ is selected from H, acetyl, *tert*-butyryl, hexanoyl, 2-methylhexanoyl, capryloyl, decanoyl, lauroyl, myristoyl, palmitoyl, stearoyl, oleoyl or linoleoyl;
wherein, R₁ is selected from H, acetyl, lauroyl, myristoyl or palmitoyl;
optionally, R₁ is H or acetyl.

Further, R₄ and R₅ are independently selected from H, methyl, ethyl, hexyl, dodecyl or hexadecyl;
wherein, R₄ is H and R₅ is selected from H, methyl, ethyl, hexyl, dodecyl or hexadecyl;
optionally, R₂ is -OH or -NH₂.

Further, X₅ is a bond, and X₇ is a bond.

Further, the peptide represented by Formula (I), or a stereoisomer thereof, or a mixture of the stereoisomers thereof, or a cosmetically acceptable salt thereof, or a pharmaceutically acceptable salt thereof, is selected from the following peptides (1)-(48):
(1) H-Val-Lys-Val-Leu-Gln-Trp-OH;
(2) H-Val-Lys-Val-Leu-Gln-Trp-NH₂;
(3) Ac-Val-Lys-Val-Leu-Gln-Trp-OH;
(4) Ac-Val-Lys-Val-Leu-Gln-Trp-NH₂;
(5) H-Ser-Met-Gly-Ala-Leu-Ala-OH;
(6) H-Ser-Met-Gly-Ala-Leu-Ala-NH₂;
(7) Ac-Ser-Met-Gly-Ala-Leu-Ala-OH;
(8) Ac-Ser-Met-Gly-Ala-Leu-Ala-NH₂;
(9) Ac-Val-His-Pro-Leu-Gln-Trp-OH;
(10) Ac-Val-His-Pro-Leu-Gln-Trp-NH₂;
(11) Ac-Ser-Arg-Leu-Pro-Leu-Trp-OH;
(12) Ac-Ser-Arg-Leu-Pro-Leu-Trp-NH₂;
(13) Ac-Trp-Lys-Val-Pro-Gln-Ala-OH;
(14) Ac-Trp-Lys-Val-Pro-Gln-Ala-NH₂;
(15) Ac-Val-Arg-Leu-Gly-Phe-Gln-His-Trp-OH;
(16) Ac-Val-Arg-Leu-Gly-Phe-Gln-His-Trp-NH₂;
(17) Ac-Val-His-Pro-Leu-Phe-Gln-His-Trp-OH;
(18) Ac-Val-His-Pro-Leu-Phe-Gln-His-Trp-NH₂;
(19) Ac-Val-Ala-Val-Leu-Phe-Gln-Arg-Trp-OH;
(20) Ac-Val-Ala-Val-Leu-Phe-Gln-Arg-Trp-NH₂;
(21) Ac-Val-Met-Gly-Pro-Trp-Leu-Lys-Ala-OH;
(22) Ac-Val-Met-Gly-Pro-Trp-Leu-Lys-Ala-NH₂;
(23) Ac-Val-Met-Ala-Pro-Tyr-Leu-Lys-Ala-OH;
(24) Ac-Val-Met-Ala-Pro-Tyr-Leu-Lys-Ala-NH₂;
(25) Ac-Ser-Ala-Val-Pro-Trp-Leu-Arg-Trp-OH;
(26) Ac-Ser-Ala-Val-Pro-Trp-Leu-Arg-Trp-NH₂;
(27) Ac-Ser-Met-Ala-Gly-Trp-Gln-His-Ala-OH;
(28) Ac-Ser-Met-Ala-Gly-Trp-Gln-His-Ala-NH₂;
(29) H-Trp-Ala-Ala-Pro-Phe-Leu-Arg-Ala-OH;
(30) H-Trp-Ala-Ala-Pro-Phe-Leu-Arg-Ala-NH₂;
(31) Ac-Trp-Ala-Ala-Pro-Phe-Leu-Arg-Ala-OH;
(32) Ac-Trp-Ala-Ala-Pro-Phe-Leu-Arg-Ala-NH₂;
(33) H-Trp-Lys-Ala-Leu-Phe-Gln-Arg-Trp-OH;
(34) H-Trp-Lys-Gly-Pro-Tyr-Gln-Met-Trp-OH;
(35) Ac-Met-Leu-Leu-Leu-Ala-Asp-NH₂;
(36) Ac-Val-Asn-Gln-Leu-Val-Ala-NH₂;
(37) Ac-Met-Leu-Asp-Phe-Leu-Ala-NH₂;
(38) Ac-Leu-Trp-Phe-Val-Phe-Lys-NH₂;
(39) Ac-Glu-Thr-Gly-Trp-Ala-Ala-NH₂;
(40) Ac-Ala-Pro-Phe-Leu-Arg-Ala-NH₂;
(41) Ac-Pro-Gly-Asp-Ala-Tyr-Gly-NH₂;
(42) Ac-Tyr-Gly-Asp-Ala-Gly-Tyr-NH₂;
(43) Ac-Ser-Phe-Ser-Asn-Gln-Met-NH₂;
(44) Ac-Val-Asp-Cys-Ala-Asn-Lys-NH₂;
(45) Ac-Val-Phe-Ala-Phe-Leu-Tyr-NH₂;
(46) Ac-Ser-Ser-Ala-Trp-Ala-Lys-NH₂;
(47) Ac-Thr-Cys-Lys-Glu-Leu-Arg-NH₂;
(48) Ac-Leu-Asn-Leu-Val-Leu-Trp-NH₂.

Further, the peptide, or a stereoisomer thereof, or a mixture of the stereoisomers thereof, or a cosmetically acceptable salt thereof, or a pharmaceutically acceptable salt thereof, is selected from Ac-Val-Lys-Val-Leu-Gln-Trp-NH₂, Ac-Ser-Met-Gly-Ala-Leu-Ala-NH₂, or Ac-Trp-Ala-Ala-Pro-Phe-Leu-Arg-Ala-NH₂.

The peptide represented by Formula (I) of the present application may be present as a stereoisomer or a mixture of stereoisomers; for example, these amino acids comprised therein may have an L- or D-configuration, or be each independently racemic. Thus, it is possible to obtain isomeric mixtures as well as racemic mixtures or diastereomeric mixtures, or pure diastereomers or enantiomers, depending on the number of asymmetric carbons and what isomers or isomeric mixtures are present. Preferred structures of the peptide represented by Formula (I) of the present application are pure isomers, i.e., enantiomers or diastereomers.

For example, when -Phe- is mentioned in the present application, it should be understood that - Phe- is selected from -L-Phe-, -D-Phe-, or a mixture of both, and is racemic or non-racemic. The preparation methods described in this document enable a person of ordinary skill in the art to obtain each stereoisomer of the peptide of the present application by selecting an amino acid having the correct configuration.

The present application further includes all suitable isotopic variants of the peptide represented by Formula (I). These isotopic variants of the peptide in the present application are here understood as indicating compounds that: at least one atom in the peptide of the present application is replaced with another atom of the same atomic number, but the atomic mass of the other atom is different from the atomic mass commonly or mainly present in nature. Examples of isotopes that can be incorporated into the peptide of the present application are those of hydrogen, carbon, nitrogen, oxygen, or sulfur, such as ²H (deuterium), ³H (tritium), ¹³C, ¹⁴C, ¹⁵N, ¹⁷O, ¹⁸O, ³³S, ³⁴S, ³⁵S or ³⁶S. The specific isotopic variants of the peptide in the present application (especially those in which one or more radioactive isotopes have been incorporated) may be advantageous, for example, in examining the mechanism of action in vivo or the distribution of active compounds; and due to its relatively simple preparation and detectability, compounds labeled with ³H or ¹⁴C isotopes are particularly suitable for this purpose. In addition, due to the stronger metabolic stability of compounds, the incorporation of isotopes (such as deuterium) can produce specific therapeutic benefits, such as prolonging the half-life in vivo or reducing the required active dosage; therefore, in some cases, such modifications of the peptide of the present application may also constitute preferred embodiments of the present application. Isotope variants of the peptide of the present application can be prepared by using methods known to those skilled in the art, such as by using the methods described further below and the methods described in the embodiments, by using corresponding isotopic modifications of the respective reagents and/or starting substances.

In addition, the present application further includes prodrugs of the peptide of the present application. The term "prodrug" used herein refers to the compounds which may be biologically active or inactive themselves but react (such as, be metabolized or hydrolyzed) during their residence time in the body to generate the peptide of the present application.

The term "a cosmetically acceptable salt or pharmaceutically acceptable salt" refers to a salt approved for use in animals, and more specifically in humans, including a metal salt of the peptide represented by Formula (I), wherein the metal includes, but is not limited to, lithium, sodium, potassium, calcium, magnesium, manganese, copper, zinc or aluminum, etc.; a salt formed from the peptide represented by Formula (I) and an organic alkali, wherein the organic alkali includes ethylamine, diethylamine, ethylenediamine, ethanolamine, diethanolamine, arginine, lysine, histidine or piperazine; a salt formed from the peptide represented by Formula (I) and an inorganic acid or an organic acid, wherein the organic acid includes, but is not limited to, acetic acid, citric acid, lactic acid, malonic acid, maleic acid, tartaric acid, fumaric acid, benzoic acid, aspartic acid, glutamic acid, succinic acid, oleic acid, trifluoroacetic acid, oxalic acid, pamoic acid, or gluconic acid, etc.; and the inorganic acid includes, but is not limited to, hydrochloric acid, sulfuric acid, boric acid or carbonic acid.

The synthesis of the peptide represented by Formula (I) of the present application, or a stereoisomer thereof, or a cosmetically acceptable salt thereof, or a pharmaceutically acceptable salt thereof can be carried out according to conventional methods known in the prior art, such as solid-phase synthesis, liquid-phase synthesis and a method of combining solid-phase and liquid-phase, as well as by biotechnological methods aimed at producing the desired sequence, or by controlled hydrolysis of animal-, fungal-, or plant-derived proteins.

For example, a method for obtaining a peptide represented by Formula (I) comprises the following steps:
- coupling an amino acid having a protected N-terminal and a free C-terminal to an amino acid having a free N-terminal and a C-terminal that is protected or bound to a solid carrier;
- removing the N-terminal protecting group;
- repeating the coupling order and removing the N-terminal protecting group until the desired peptide sequence is obtained; and
- removing the C-terminal protecting group or cleaving from the solid carrier.

Preferably, the C-terminal is bound to a solid carrier and the method is carried out on the solid phase, including coupling an amino acid having a protected N-terminal and a free C-terminal to an amino acid having a free N-terminal and a C-terminal that is bound to a polymer carrier; removing the N-terminal protecting group; repeating this order by the required times thereby obtaining a peptide having the desired length, followed by cleaving the synthesized peptide from the initial polymer carrier.

The functional groups of the side chains of the amino acids remain fully protected with temporary or permanent protecting groups throughout the synthesis and can be deprotected simultaneously or orthogonally in the process of cleaving the peptide from the polymer carrier.

Alternatively, solid phase synthesis can be performed by a convergent strategy in which a dipeptide or tripeptide is coupled to a polymer support or to a dipeptide or amino acid previously bound to a polymer support.

The N-terminal and C-terminal are deprotected and/or the peptide is cleaved from the polymer support in a non-determined order using standard conditions and methods known in the art, and subsequently the terminal functional groups can be modified. Optional modifications to N-terminal and C-terminal can be made to the peptide represented by Formula (I) bound to the polymer support, or optional modifications to N-terminal and C-terminal can be made after the peptide has been cleaved from the polymer support.

Another aspect of the present application provides a cosmetic or pharmaceutical composition, which comprises an effective amount of the peptide represented by Formula (I) above, or a stereoisomer thereof, or a mixture of the stereoisomers thereof, or a cosmetically acceptable salt thereof, or a pharmaceutically acceptable salt thereof, and at least one excipient and optionally a cosmetically or pharmaceutically acceptable adjuvant.

Specifically, the adjuvant is selected from a collagen synthesis stimulator, an agent that modulates the PGC-1α synthesis, an agent that modulates the activity of PPARγ, an agent that increases or decreases a triglyceride content in adipocytes, an agent that stimulates or delays adipocyte differentiation, a lipolytic agent or an agent that simulates lipolysis, a fat clearing agent, an adipogenic agent, an inhibitor against acetylcholine receptor aggregation, an agent that inhibits muscle contraction, an anticholinergic agent, an elastase inhibitor, a matrix metalloproteinases inhibitor, a melanin synthesis stimulator or inhibitor, a whitening or decoloring agent, a pigmentation promoter, a self-tanning agent, an antiaging agent, a NO-synthase inhibitor, a 5α-reductase inhibitor, an inhibitor against lysyl hydroxylase and/or prolyl hydroxylase, an antioxidant, a free radical scavenger and/or an anti-air pollution agent, a reactive carbonyl species scavenger, an anti-glycation agent, an antihistamine, an antiviral agent, an antiparasitic agent, an emulsifier, an emollient, an organic solvent, a liquid propellant, a skin conditioner, a humectant, a moisture retaining substance, an alpha-hydroxy acid, a beta-hydroxy acid, a moisturizer, an epidermal hydrolase, a vitamin, an amino acid, a protein, a pigment or colorant, a dye, a biopolymer, a gelling polymer, a thickener, a surfactant, a softener, an adhesive, a preservative, an anti-wrinkle agent, an agent that can reduce or treat under-eye bags, an exfoliant, a cuticle stripping agent, a keratolytic agent, an antimicrobial, an antifungal, a fungistat, a bactericide, a bacteriostat, an agent that stimulates the synthesis of dermal or epidermal macromolecules and/or inhibits or prevents the degradation thereof, an agent that stimulates elastin synthesis, an agent that stimulates decorin synthesis, an agent that stimulates laminin synthesis, an agent that stimulates defensin synthesis, an agent that stimulates chaperone protein synthesis, an agent that stimulates cAMP synthesis, a heat shock protein, an agent that stimulates the HSP70 synthesis, an agent that stimulates the synthesis of a heat shock protein, an agent that stimulates the synthesis of hyaluronic acid, an agent that stimulates fibronectin synthesis, an agent that stimulates deacetylase synthesis, an agent that stimulates the synthesis of a lipid and a stratum corneum component, ceramide, a fatty acid, an agent that inhibits collagen degradation, an agent that inhibits elastin degradation, an agent that inhibits serine protease, an agent that stimulates fibroblast proliferation, an agent that stimulates keratinocyte proliferation, an agent that stimulates adipocyte proliferation, an agent that stimulates melanocyte proliferation, an agent that stimulates keratinocyte differentiation, an agent that inhibits acetylcholinesterase, a skin relaxant, an agent that stimulates glycosaminoglycan synthesis, an anti-hyperkeratosis agent, an comedolytic agent, an antipsoriatic agent, an anti-dermatitis agent, an anti-eczema agent, a DNA repair agent, a DNA protecting agent, a stabilizer, an antipruritic agent, an agent for the treatment and/or care of sensitive skin, a hardening agent, a tightening agent, a restructuring agent, an anti-stretching agent, an adhesive, an agent that modulates sebum production, an antiperspirant, an agent that stimulates healing, an agent that assists healing, an agent that stimulates re-epithelialization, an agent that assists re-epithelialization, a cytokine growth factor, a sedative, an anti-inflammatory agent, an anesthetic agent, an agent acting on capillary circulation and/or microcirculation, an agent that stimulates angiogenesis, an agent that inhibits vascular permeability, a venotonic agent, an agent acting on cell metabolism, an agent for improving dermis-epidermis connection, an agent that induces hair growth, a hair growth inhibiting or retarding agent, a fragrance, a chelating agent, a plant extract, an essential oil, a marine extract, an agents derived from a biological fermentation process, an inorganic salt, a cell extract, a sunscreen, and an effective organic or inorganic photoprotectant against A and/or B UV rays, or mixtures thereof.

Further, a preparation of the cosmetic or pharmaceutical composition is selected from a cream, an oil, milk, a balm, a foam, a lotion, a gel, a liniment, sera, a soap, a shampoo, a hair conditioner, a serum, an ointment, a mousse, a pomade, a powder, a bar, a pencil, a spray, an aerosol, a capsule, a tablet, a granule, a chewing gum, a solution, a suspension, an emulsion, a syrup, an elixir, a polysaccharide film, a jelly or a gelatin;
specifically, the capsule includes a soft capsule, a hard capsule, and optionally a gelatin capsule; and the tablet includes a sugar-coated tablet.

The peptides of the present application have variable solubilities in water depending on the nature of their sequences or any potential modifications in the N-terminal and/or C-terminal. The peptide of the present application may therefore be incorporated into a composition via an aqueous solution, and those that are insoluble in water may be soluble in cosmetically or pharmaceutically acceptable conventional solvents, examples of the solvents include but are not limited to ethanol, propanol, isopropanol, propylene glycol, glycerin, butylene glycol or polyethylene glycol or any combination thereof.

The cosmetically or pharmaceutically effective amount of the peptide of the present application to be administered, as well as dosages thereof, will depend on many factors including age, the state of a patient, the severity of a condition or disease, the route and frequency of administration, and specific properties of the peptide to be used.

"A cosmetically or pharmaceutically effective amount" refers to an amount of one or more peptides of the present application that is nontoxic but sufficient to provide the desired effects. The peptide of the present application is used in the cosmetic composition or pharmaceutical composition of the present application at a cosmetically or pharmaceutically effective concentration to obtain the desired effects; In one preferred form, relative to the total weight of the composition, the concentration is between 0.00000001% (by weight) and 20% (by weight), preferably between 0.000001% (by weight) and 15% (by weight), more preferably between 0.0001% (by weight) and 10 % (by weight), and even more preferably between 0.0001% (by weight) and 5% (by weight).

Another aspect of the present application provides a cosmetically or pharmaceutically acceptable delivery system or sustained release system, to achieve better penetration of the active ingredient and/or to improve pharmacokinetic and pharmacodynamic properties thereof, which comprises an effective amount of the peptide represented by Formula (I) above, or a stereoisomer thereof, or a mixture of the stereoisomers thereof, or a cosmetically acceptable salt thereof, or a pharmaceutically acceptable salt thereof, or the cosmetic or pharmaceutical composition above.

The term "delivery system" refers to a diluent, adjuvant, excipient or carrier administered with the peptide of the present application, selected from water, oils or surfactants, including those petroleum-derived, animal-derived, plant-derived, or synthesis-derived. Examples include but are not limited to peanut oil, soybean oil, mineral oil, sesame oil, castor oil, polysorbate, sorbitan ester, ether sulfate, sulfate, betaine, glucoside, maltoside, fatty alcohol, nonoxynol, poloxamer, polyoxyethylene, macrogol, dextrose, glycerin, digitonin and the like. Diluents that can be used in the different delivery systems in which the peptide of the present application can be administered are known to those of ordinary skill in the art.

The term "sustained release" is used in the conventional sense to refer to a delivery system that provides a gradual release of a compound over a period of time, and preferably, but not necessarily, a relatively constant level of compound release over the entire period of time.

Examples of the delivery system or sustained release system include a liposome, an oleosome, a nonionic surfactant liposome vesicle, an ethosome, a millicapsule, a microcapsule, a nanocapsule, a nanostructured lipid carrier, a sponge, a cyclodextrin, a lipoid vesicle, a micelle, a millisphere, a microsphere, a nanosphere, a liposphere, a microemulsion, a nanoemulsion, a milliparticle, a microparticle or a nanoparticle. The preferred delivery system or sustained release system is a liposome and a microemulsion, more preferably a water-in-oil microemulsion having an internal structure of reverse micelle.

The sustained release system can be prepared by methods known in the prior art and can be administered, for example, by topical or transdermal administration, including adhesive patches, non-adhesive patches, sealing patches, and microelectronic patches; or by systemic administration, such as but not limited to, oral or parenteral routes, including nasal, rectal, subcutaneous implantation or injection, or direct implantation or injection to specific body parts. Preferably a relatively constant amount of these peptides of the present application should be released. The amount of the peptide comprised in the sustained release system will depend, for example, on the site where the composition is to be administered, the release kinetics and duration of the peptide of the present application, and the nature of the condition, disorder and/or disease to be treated and/or cared for.

Another aspect of the present application provides use of the peptide represented by Formula (I) above, or a stereoisomer thereof, or a mixture of the stereoisomers thereof, or a cosmetically acceptable salt thereof, or a pharmaceutically acceptable salt thereof, or the above cosmetic or pharmaceutical composition, or the above cosmetically or pharmaceutically acceptable delivery system or sustained release system in the preparation of a cosmetic composition or pharmaceutical composition for treatment or care of a condition, a disorder or a disease caused by muscle contraction, wherein the disorder or the disease is dystonia.

In an embodiment of the present application, the disorder or the disease is dystonia, and specifically, the dystonia is focal dystonia; dystonia includes, but is not limited to, facial spasm, torsion dystonia, cercival dystonia or torticollis, laryngeal dystonia or spasmodic dysphonia, oromandibular dystonia, limb dystonia such as graphospasm or musician's cramp or dystonia of the feet, bruxism, hemifacial spasm, tic disorder, and/or strabismus; segmental dystonia, Meige's syndrome, multifocal dystonia, hemidystonia, dopamine-responsive dystonia, and Segawa dystonia.

Another aspect of the present application provides use of the peptide represented by Formula (I) above, or a stereoisomer thereof, or a mixture of the stereoisomers thereof, or a cosmetically acceptable salt thereof, or a pharmaceutically acceptable salt thereof, or the above cosmetic or pharmaceutical composition, or the above cosmetically or pharmaceutically acceptable delivery system or sustained release system in the preparation of a cosmetic composition or pharmaceutical composition for treatment, prevention or repair of skin aging or photoaging, wherein the treatment, prevention or repair of skin aging or photoaging is to reduce, prevent or treat facial wrinkles.

In order to facilitate understanding of the present application, the meanings of some terms and expressions used in the present application are explained as follows.

In the present application, the term "skin" should be understood to be the layers which comprise it, from the uppermost layer or stratum corneum to the lowermost layer or subcutaneous tissue, both inclusive. These layers are composed of different types of cells such as keratinocytes, fibroblasts, melanocytes and/or adipocytes, among others. In the present application, the term "skin" includes the scalp.

The term "treatment" refers to the administration of a peptide according to the present application to alleviate or eliminate a disease or disorder, or reduce or eliminate one or more symptoms associated with this disease or disorder. The term "treatment" also covers the ability to alleviate or eliminate the physiological consequences of the disease or disorder.

The term "care" includes the prevention of a disease and/or disorder.

The term "prevention" refers to the ability of a peptide of the present application to prevent, delay or hinder the appearance or development of a disease or disorder before its appearance.

The term "aging" refers to changes experienced by the skin with age (natural aging) or through exposure to the sun (photoaging) or to environmental contaminants such as chemical fouling or contaminants, tobacco smoke, including all the externally visible and/or perceptible changes through touch, such as but not limited to, the development of discontinuities on the skin (e.g., wrinkles, fine lines, expression lines, stretch lines, striae, furrows, irregularities or roughness, increased pore size, loss of moisture, loss of elasticity, loss of firmness, loss of smoothness, loss of the capacity to recover from deformation, loss of resilience), sagging of the skin (e.g., sagging cheeks, bags under the eyes, or double chins, etc.), changes in skin color (such as scarring, redness, under-eye bags, or areas of hyperpigmentation like age spots or freckles, etc.), abnormal differentiation, hyperkeratinization, elastosis, keratosis, hair loss, orange peel skin, loss of collagen structure, and other histological changes in the stratum corneum, dermis, epidermis, vasculature (e.g., the appearance of spider veins or telangiectasias), or those tissues adjacent to the skin.

The term "photoaging" refers to premature aging of the skin due to long-term exposure of the skin to UV radiation, exhibiting the same physiological characteristics as natural aging, such as but not limited to, looseness, sagging, color changes or irregularities in the pigmentation, abnormal and/or excessive keratinization.

In this specification, the abbreviations used for amino acids follow the rules specified by the IUPAC-IUB Commission of Biochemical Nomenclature in the European Journal of Biochemistry (Eur. J. Biochem. 1984, 138:9-37).

Therefore, for example, Lys represents NH₂-CH(CH₂CH₂CH₂CH₂NH₂)-COOH, Lys- represents NH₂-CH(CH₂CH₂CH₂CH₂NH₂)-CO-, -Lys represents -NH-CH(CH₂CH₂CH₂CH₂NH₂)-COOH, and - Lys- represents -NH-CH(CH₂CH₂CH₂CH₂NH₂)-CO-. Thus, a hyphen representing a peptide bond removes OH in amino acid 1-carboxyl (herein represented in the conventional non-ionized form) when located to the right of the symbol, and removes H in amino acid 2-amino when located to the left of the symbol.

The abbreviation "Ac-" is used in the present application to represent acetyl (CH₃-CO-); Ser: serine; Gly: glycine; Ala: alanine; Lys: lysine; Trp: tryptophan; Val: valine; Leu: leucine; Gln: glutamine; Met: methionine; His: histidine; Arg: arginine; Pro: proline; Phe: phenylalanine; Tyr: tyrosine.

The present application has the following advantages and effects.

The polypeptide in the present application is obtained by artificial designing and features convenient synthesis. By interacting with the synaptic vesicle associated membrane protein (VAMP2), it regulates the formation of SNARE complexes, thereby interfering with the release of synaptic neurotransmitters, inhibiting muscle contraction, improving wrinkles on the skin surface, reducing wrinkle depth, and achieving wrinkle removal effects. Therefore, the polypeptide can be used in the cosmetics or pharmaceutical fields to improve skin aging or photoaging, and to treat or care for conditions, disorders, or diseases caused by muscle contraction.

### DESCRIPTION OF THE DRAWINGS

To describe the technical solutions of the present application more clearly, the following briefly describes the accompanying drawings required for describing the present application. Apparently, the accompanying drawings in the following description show merely some embodiments of the present application, and those of ordinary skill in the art may still obtain other accompanying drawings from these accompanying drawings without creative efforts.
FIG. 1 is a graph showing the mass spectrum of the peptide (4) Ac-Val-Lys-Val-Leu-Gln-Trp-NH₂ (molecular formula C₄₀H₆₄N₁₀O₈), in which the mass-to-charge ratio (m/z) of the [M+H]⁺ excimer ion peak is 813.5879, and the molecular weight measured by mass spectrometry is 812.59;
FIG. 2 is a graph showing the mass spectrum of the peptide (8) Ac-Ser-Met-Gly-Ala-Leu-Ala-NH₂ (molecular formula C₂₄H₄₃N₇O₈S), in which the mass-to-charge ratio (m/z) of the [M+Na]⁺ adduct ion peak is 612.3438, and the molecular weight measured by mass spectrometry is 589.34;
FIG. 3 is a graph showing the mass spectrum of the peptide (32) Ac-Trp-Ala-Ala-Pro-Phe-Leu-Arg-Ala-NH₂ (molecular formula C₄₈H₆₉N₁₃O₉), in which the mass-to-charge ratio (m/z) of the [M+H]⁺ excimer ion peak is 972.6496, and the molecular weight measured by mass spectrometry is 971.65;
FIG. 4 is a graph showing the results of the muscle contraction inhibition experiment on the test samples.

### SPECIFIC IMPLEMENTATIONS

To make the foregoing objects, features and advantages of the present application more apparent and comprehensible, a detailed description is further made to the present application below with reference to the accompanying drawings and the embodiments. Apparently, the described embodiments are only some embodiments rather than all the embodiments of the present application. All other embodiments obtained by those of ordinary skill in the art based on the embodiments of the present application without creative efforts fall within the protection scope of the present application.

The core conception of the present application is that a biomimetic peptide is obtained based on Synaptophysin, and the peptide can interfere with the interaction between Synaptophysin and VAMP2, thereby inhibiting the formation of SNARE complexes and the release of neurotransmitters, thus suppressing muscle contraction. The polypeptide has the following general formula: R₁-X₁-X₂-X₃-X₄-X₅-X₆-X₇-X₈-R₂; the present application relates to a peptide, a stereoisomer thereof, a mixture of the stereoisomers, or a cosmetically acceptable salt thereof, or a pharmaceutically acceptable salt thereof, or a cosmetic composition or a pharmaceutical composition thereof, and a use thereof in the preparation of a cosmetic composition or pharmaceutical composition for improving skin aging or photoaging, and treating or caring for a condition, a disorder or a disease caused by muscle contraction.

It should be noted that, in the present application, the abbreviations used for amino acids follow the rules specified by the Commission of Biochemical Nomenclature of IUPAC-IUB in Eur. J. Biochem. (1984) 138: 9-37 and J. Chem (1989) 264: 633-673.

Amide Resin: a starting resin for polypeptide synthesis (cross-linking degree 1%, substitution degree 1.72 mmol/g, grain size 100-200 mesh); Fmoc-Linker: 4-[(2,4-dimethoxyphenyl)(Fmoc-amino)methyl]phenoxyacetic acid; Ac₂O: acetic anhydride; DMF: N,N-dimethylformamide; DIPEA: diisopropylethylamine; DIC: diisopropylcarbodiimide; piperidine: piperidine; HOBt: 1-hydroxybenzotriazole; TFA: trifluoroacetic acid; TIS: triisopropylsilane; EDT: 1,2-ethanedithiol; Ser: serine; Gly: glycine; Ala: alanine; Lys: lysine; Trp: tryptophan; Val: valine; Leu: leucine; Gln: glutamine; Met: methionine; Arg: arginine; Pro: proline; Phe: phenylalanine; Fmoc: 9-fluorenylmethoxycarbonyl; Boc: *tert*-butoxycarbonyl; tBu: *tert*-butyl; Trt: trityl; Pbf: 2,2,4,6,7-pentamethyldihydrobenzofuran-5-sulfonyl.

### EXAMPLE 1 Computer simulation

In this experiment, candidate peptides were screened out using computer simulation methods, the candidate peptides had affinity or specificity for the interaction area between Synaptophysin and VAMP2, and thus, they can interfere with the interaction or binding between Synaptophysin and VAMP2, thereby affecting the formation of SNARE complexes.

Firstly, different lengths of amino acid residue fragments were extracted from the full sequence of Synaptophysin serving as the target protein, so that overlapping peptide libraries with peptide chain lengths of 5, 6, 7, and 8 AAs respectively were constructed. Due to the helical bundle structure of VAMP2 in SNARE complexes, α-helic had a significant impact on the affinity between polypeptides and VAMP2, and thus, the α-helic properties of the polypeptides were analyzed using computers.

Based on the computer simulation results of the α-helic properties of polypeptides, 27 polypeptides were preliminarily screened out, as shown in Table 1.

**Table 1 Polypeptides preliminary screened out using computer simulation**

| Sequence | Predicted results |
|---|---|
| Ac-TGWAA-NH₂ | 0.33 |
| Ac-PFLRA-NH₂ | 0.45 |
| Ac-GDAYG-NH₂ | 0.15 |
| Ac-GDAYG-NH₂ | 0.15 |
| Ac-PQDSY-NH₂ | 0.12 |
| Ac-MLLLAD-NH₂ | 0.69 |
| Ac-VNQLVA-NH₂ | 0.52 |
| Ac-VKVLQW-NH₂ | 0.60 |
| Ac-MLDFLA-NH₂ | 1.04 |
| Ac-LWFVFK-NH₂ | 0.60 |
| Ac-ETGWAA-NH₂ | 0.31 |
| Ac-APFLRA-NH₂ | 0.15 |
| Ac-PGDAYG-NH₂ | 0.20 |
| Ac-YGDAGY-NH₂ | 0.12 |
| Ac-SFSNQM-NH₂ | 0.15 |
| Ac-VDCANK-NH₂ | 0.69 |
| Ac-VFAFLY-NH₂ | 1.07 |
| Ac-SMGALA-NH₂ | 1.55 |
| Ac-SSAWAK-NH₂ | 1.39 |
| Ac-TCKELR-NH₂ | 1.02 |
| Ac-LNLVLW-NH₂ | 1.38 |
| Ac-KETGWAA-NH₂ | 0.26 |
| Ac-DAYGDAG-NH₂ | 0.14 |
| Ac-DYGQQGY-NH₂ | 0.11 |
| Ac-WAAPFLRA-NH₂ | 0.11 |
| Ac-FKETGWAA-NH₂ | 0.19 |
| Ac-DAYGDAGY-NH₂ | 0.14 |

In order to further evaluate the binding ability of polypeptides with VAMP2, VAMP2 before participating in the formation of SNARE complexes was selected for study. The affinity of the aforementioned polypeptides with VAMP2 was calculated using molecular docking and molecular dynamics so that the best candidate peptides were screened out for which further activity validation was considered. The best candidate peptides are shown in Table 2 below.

**Table 2 Best candidate polypeptides screened out by computer simulation**

| Sequence | Binding energy (kcal/mol) |
|---|---|
| Ac-VKVLQW-NH₂ | -21.0010 |
| Ac-SMGALA-NH₂ | -11.2576 |
| Ac-WAAPFLRA-NH₂ | -15.5384 |

The results showed that the peptide of the present application can bind well with VAMP2 so that it interferes with the formation of SNARE complexes and can be used to inhibit muscle contraction.

### EXAMPLE 2

### Preparation of Ac-Val-Lys-Val-Leu-Gln-Trp-NH₂

### 2.1 Preparation of Fmoc-Linker-Amide Resin

5 g of Amide Resin was weighed and added to a solid-phase synthesis reaction column, and swollen with DMF. The resin was washed and the solvent was removed.

7 g of Fmoc-Linker and 2.10 g of HOBt were weighed in a dry conical flask. After DMF solvent was used for dissolution, the solution was cooled in an ice-water bath for 10 min, and DIC was added for activation for 10 min while avoiding water vapor.

The activated Fmoc-Linker was added to the swollen resin to react for 2.5 h, and then the reaction solution was removed. The resin was washed and the solvent was removed.

Ac₂O and DIPEA were further added for capping for 1.5 h. The resin was washed and the solvent was removed.

### 2.2 Fmoc deprotection

Fmoc-Linker-Amide Resin was Fmoc-deprotected twice using 20% piperidine/DMF, each time for 10 min. A sample was taken for the K-test, showing a color of dark blue. The resin was washed with DMF 7 times, and the solvent was removed.

### 2.3 Feeding reaction

5.3 g of Fmoc-Trp(Boc)-OH and 1.8 g of HOBt were weighed and added into a dry conical flask, and the resultant was dissolved by adding DMF and then sealed and placed in a refrigerator at -18°C for 30 min. 1.65 g of DIC was added for activation for 3 min while avoiding water vapor. The activated amino acid was added to the deprotected resin to react for 2 h. The reaction solution was removed. The K-test showed that the resin was colorless and transparent, indicating that the reaction was complete.

The N-terminal Fmoc group was deprotected, and in the presence of 1.8 g HOBt and 1.65 g DIC, 7.3 g of the activated Fmoc-Gln(Trt)-OH was coupled to the peptidyl resin using DMF as solvent, and the reaction lasted 2 h. The resins were then washed and repeatedly subjected to the deprotection treatment of the Fmoc group so as to couple to the next amino acid. In each coupling, in the presence of 1.8 g HOBt and 1.65 g DIC, 4.2 g of Fmoc-Leu-OH; 4.0 g of Fmoc-Val-OH, 5.6 g of Fmoc-Lys(Boc)-OH and subsequent 4.0 g of Fmoc-Val-OH were sequentially coupled using DMF as solvent; after the completion of the reaction, the resin was washed and the solvent was removed.

The N-terminal Fmoc group of the peptidyl resin was deprotected twice using 20% piperidine/DMF, each time for 10 min, and a sample was taken for the K-test, showing a color of dark blue. The resin was washed with DMF 6 times, and the solvent was removed.

In the presence of DIPEA, 1.9 g of Ac₂O was coupled to the peptidyl resin using DMF as solvent, the reaction lasted 1.5 h, then the resin was washed, and the solvent was removed. After shrinkage drying, 12 g of Ac-Val-Lys(Boc)-Val-Leu-Gln(Trt)-Trp(Boc)-Linker-Amide Resin was obtained.

### 2.4 Cleavage

54 mL of TFA, 1.5 mL of TIS, 1.5 mL of EDT, 1.5 mL of thioanisole, and 1.5 mL of water were measured, mixed and stirred uniformly to obtain a lysis buffer, which was sealed and placed in a refrigerator at -18°C for subsequent use; and isopropyl ether was placed in a refrigerator at -18°C for subsequent use.

12 g of Ac-Val-Lys(Boc)-Val-Leu-Gln(Trt)-Trp(Boc)-Linker-Amide Resin was weighed and added to a round-bottom flask, the above-mentioned frozen lysis buffer was added, and the mixture was stirred and reacted for 2 h. The mixture was filtered and the filtrate was collected and concentrated to 10 mL, then isopropyl ether was added, stirred, centrifuged and washed 6 times until the pH value was 3-4, and the resultant was vacuum dried to obtain 4.5 g of Ac-Val-Lys-Val-Leu-Gln-Trp-NH₂ crude peptide.

### 2.5 Purification

4.5 g of crude peptide was weighed and dissolved in pure water, the resultant was concentrated and then completely dissolved by adding methanol thereto, and the mixture was filtered with a microporous filter membrane having an aperture of 0.22 µm to afford a clear and transparent solution. The solution was subjected to reversed-phase HPLC purification, and the purification gradient is shown in the following table:

| Time (min) | Flow rate (mL/min) | A% (0.1% acetic acid + acetonitrile) | B% (0.1% acetic acid + pure water) |
|---|---|---|---|
| 0 | 40 | 10 | 90 |
| 15 | 40 | 30 | 70 |
| 50 | 40 | 45 | 55 |

The filtered sample was injected and purified, the fractions were collected, concentrated and freeze-dried to afford the peptide (4) Ac-Val-Lys-Val-Leu-Gln-Trp-NH₂ having a purity of 98%.

### EXAMPLE 3

### Preparation of Ac-Ser-Met-Gly-Ala-Leu-Ala-NH₂

### 3.1 Preparation of Fmoc-Linker-Amide Resin

5 g of Amide Resin was weighed and added to a solid-phase synthesis reaction column, and swollen with DMF. The resin was washed and the solvent was removed.

6.9 g of Fmoc-Linker and 2.0 g of HOBt were weighed in a dry conical flask. After DMF solvent was used for dissolution, the solution was cooled in an ice-water bath for 10 min, and DIC was added for activation for 10 min while avoiding water vapor.

The activated Fmoc-Linker was added to the swollen resin to react for 2.5 h, and then the reaction solution was removed. The resin was washed and the solvent was removed.

Ac₂O and DIPEA were further added for capping for 1.5 h. The resin was washed and the solvent was removed.

### 3.2 Fmoc deprotection

Fmoc-Linker-Amide Resin was Fmoc-deprotected twice using 20% piperidine/DMF, each time for 10 min, and a sample was taken for the K-test, showing a color of dark blue. The resin was washed with DMF 8 times, and the solvent was removed.

### 3.3 Feeding reaction

3.1 g of Fmoc-Ala-OH and 1.8 g of HOBt were weighed and added into a dry conical flask, the mixture was dissolved by adding DMF thereto, and the resultant was sealed and placed in a refrigerator at -18°C for 30 min. 1.65 g DIC was added for activation for 3 min while avoiding water vapor. The activated amino acid was added to the deprotected resin to react for 2 h. The reaction solution was removed. The K-test showed that the resin was colorless and transparent, indicating that the reaction was complete.

The N-terminal Fmoc group was deprotected, and in the presence of 1.8 g HOBt and 1.65 g DIC, 4.2 g of the activated Fmoc-Leu-OH was coupled to the peptidyl resin using DMF as solvent, and the reaction lasted 2 h. The resins were then washed and repeatedly subjected to the deprotection treatment of the Fmoc group so as to couple to the next amino acid. In each coupling, in the presence of 1.8 g HOBt and 1.65 g DIC, 3.1 g of Fmoc-Ala-OH, 3.6 g of Fmoc-Gly-OH, 4.5 g of Fmoc-Met-OH and subsequent 4.6 g of Fmoc-Ser(tBu)-OH were sequentially coupled using DMF as solvent; After the completion of the reaction, the resin was washed and the solvent was removed.

The N-terminal Fmoc group of the peptidyl resin was deprotected twice using 20% piperidine/DMF, each time for 10 min, and a sample was taken for the K-test, showing a color of dark blue. The resin was washed with DMF 6 times, and the solvent was removed.

In the presence of DIPEA, 1.9 g of Ac₂O was coupled to the peptidyl resin using DMF as solvent, the reaction lasted 1.5 h, then the resin was washed, and the solvent was removed. After shrinkage drying, 13 g of Ac-Ser(tBu)-Met-Gly-Ala-Leu-Ala-Linker-Amide Resin was obtained.

### 3.4 Cleavage

44 mL of TFA, 1.2 mL of TIS, 1.2 mL of EDT, and 1.2 mL of water were measured, mixed and stirred uniformly to obtain a lysis buffer, which was sealed and placed in a refrigerator at -18°C for subsequent use; and isopropyl ether was placed in a refrigerator at -18°C for subsequent use.

13 g of Ac-Ser(tBu)-Met-Gly-Ala-Leu-Ala-Linker-Amide Resin was weighed and added to a round-bottom flask, the above-mentioned frozen lysis buffer was added, and the mixture was stirred and reacted for 2 h. The mixture was filtered and the filtrate was collected and concentrated to 10 mL, then isopropyl ether was added, stirred, centrifuged and washed 6 times until the pH value was 3-4, and the resultant was vacuum dried to obtain 6.9 g of Ac-Ser-Met-Gly-Ala-Leu-Ala-NH₂ crude peptide.

### 3.5 Purification

6.9 g of crude peptide was weighed and dissolved in 370 mL of pure water, and the resultant was filtered with a microporous filter membrane having an aperture of 0.22 µm to afford a clear and transparent solution. The solution was subjected to reversed-phase HPLC purification, and the purification gradient is shown in the following table:

| Time (min) | Flow rate (mL/min) | A% (0.1% acetic acid + acetonitrile) | B% (0.1% acetic acid + pure water) |
|---|---|---|---|
| 0 | 40 | 10 | 90 |
| 15 | 40 | 18 | 82 |
| 60 | 40 | 38 | 62 |

The filtered sample was injected and purified, the fractions were collected, concentrated and freeze-dried to afford the peptide (8) Ac-Ser-Met-Gly-Ala-Leu-Ala-NH₂ having a purity of 99.4%.

### EXAMPLE 4

### Preparation of Ac-Trp-Ala-Ala-Pro-Phe-Leu-Arg-Ala-NH₂

### 4.1 Preparation of Fmoc-Linker-Amide Resin

5 g of Amide Resin was weighed and added to a solid-phase synthesis reaction column, and swollen with DMF. The resin was washed and the solvent was removed.

6.9 g of Fmoc-Linker and 2.0 g of HOBt were weighed in a dry conical flask. After DMF was used for dissolution, the solution was cooled in an ice-water bath for 10 min, and DIC was added for activation for 10 min while avoiding water vapor.

The activated Fmoc-Linker was added to the swollen resin to react for 2.5 h, and then the reaction solution was removed. The resin was washed and the solvent was removed.

Ac₂O and DIPEA were further added for capping for 1.5 h. The resin was washed and the solvent was removed.

### 4.2 Fmoc deprotection

Fmoc-Linker-Amide Resin was Fmoc-deprotected twice using 20% piperidine/DMF, each time for 10 min, and a sample was taken for the K-test, showing a color of dark blue. The resin was washed with DMF 7 times, and the solvent was removed.

### 4.3 Feeding reaction

3.1 g of Fmoc-Ala-OH and 1.8 g of HOBt were weighed and added into a dry conical flask. DMF was added to dissolve the mixture, and the solution was sealed and placed in a refrigerator at - 18°C for 30 min. 1.65 g of DIC was added for activation for 3 min while avoiding water vapor. The activated amino acid was added to the deprotected resin to react for 2 h. The reaction solution was removed. The K-test showed that the resin was colorless and transparent, indicating that the reaction was complete.

The N-terminal Fmoc group was deprotected, and in the presence of 1.8 g HOBt and 1.65 g DIC, 7.6 g of the activated Fmoc-Arg(Pbf)-OH was coupled to the peptidyl resin using DMF as solvent, and the reaction lasted 2 h. The resins were then washed and repeatedly subjected to the deprotection treatment of the Fmoc group so as to couple to the next amino acid. In each coupling, in the presence of 1.8 g HOBt and 1.65 g DIC, 4.2 g of Fmoc-Leu-OH; 5.3 g of Fmoc-Phe-OH; 4.62 g of Fmoc-Pro-OH; 4.26 g of Fmoc-Ala-OH; 4.26 g of Fmoc-Ala-OH and subsequent 9.26 g of Fmoc-Trp(Boc)-OH were sequentially coupled using DMF as solvent; After the completion of the reaction, the resin was washed and the solvent was removed.

The N-terminal Fmoc group of the peptidyl resin was deprotected twice using 20% piperidine/DMF, each time for 10 min, and a sample was taken for the K-test, showing a color of dark blue. The resin was washed with DMF 6 times, and the solvent was removed.

In the presence of DIPEA, 1.9 g of Ac₂O was coupled to the peptidyl resin using DMF as solvent, the reaction lasted 1.5 h, then the resin was washed, and the solvent was removed. After shrinkage drying, 20.3 g of Ac-Trp(Boc)-Ala-Ala-Pro-Phe-Leu-Arg(Pbf)-Ala-Linker-Amide Resin was obtained.

### 4.4 Cleavage

114 mL of TFA, 3 mL of TIS, and 3 mL of water were measured, mixed and stirred uniformly to obtain a lysis buffer, which was sealed and placed in a refrigerator at -18°C for subsequent use; and isopropyl ether was placed in a refrigerator at -18°C for subsequent use.

20.3 g of Ac-Trp(Boc)-Ala-Ala-Pro-Phe-Leu-Arg(Pbf)-Ala-Linker-Amide Resin was weighed and added to a round-bottom flask, the above-mentioned frozen lysis buffer was added, and the mixture was stirred and reacted for 2 h. The mixture was filtered and the filtrate was collected and concentrated to 10 mL, then isopropyl ether was added, stirred, centrifuged and washed 6 times until the pH value was 3-4, and the resultant was vacuum dried. 9.3 g of Ac-Trp-Ala-Ala-Pro-Phe-Leu-Arg-Ala-NH₂ crude peptide was obtained.

### 4.5 Purification

9.3 g of crude peptide was weighed and dissolved in 100 mL methanol, 500 mL of pure water was added, and after the mixture was concentrated, 100 mL of methanol was supplemented. The resultant was filtered with a microporous filter membrane having an aperture of 0.22 µm to afford a clear and transparent solution. The solution was subjected to reversed-phase HPLC purification, and the purification gradient is shown in the following table:

| Time (min) | Flow rate (mL/min) | A% (0.1% acetic acid + acetonitrile) | B% (0.1% acetic acid + pure water) |
|---|---|---|---|
| 0 | 40 | 20 | 80 |
| 15 | 40 | 30 | 70 |
| 50 | 40 | 45 | 55 |

The filtered sample was injected and purified, the fractions were collected, concentrated and freeze-dried to afford the peptide (32) Ac-Trp-Ala-Ala-Pro-Phe-Leu-Arg-Ala-NH₂ having a purity of 98%.

### EXAMPLE 5

Other compounds of Formula (I) of the present application can be prepared in similar methods.

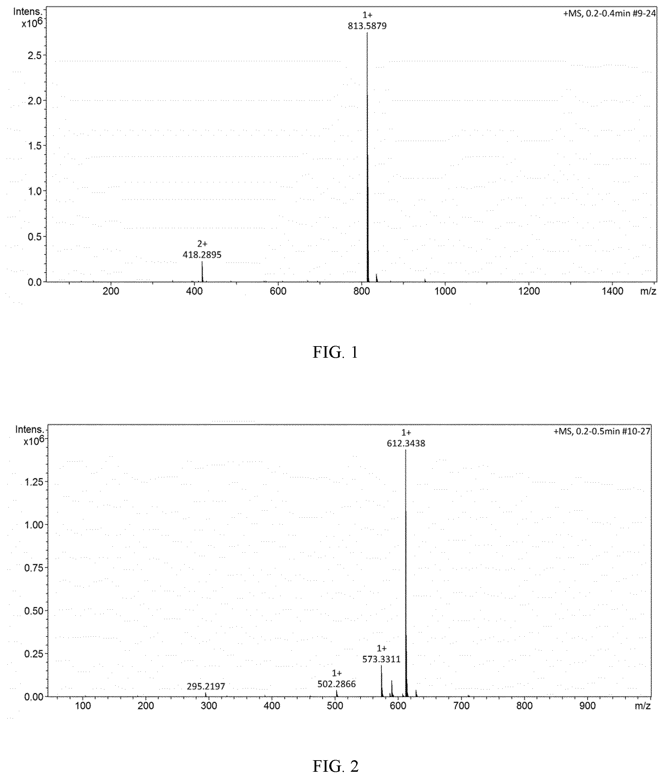

The molecular weight of the obtained peptides was determined by ESI-MS. The test results of some compounds are shown in Table 3 below and FIG. 1 to FIG. 3.

**Table 3 Results of determination of molecular weight by mass spectrometry**

| No. | Sequence | Molecular weight mass spectrometry analysis results |
|---|---|---|
| (4) | Ac-Val-Lys-Val-Leu-Gln-Trp-NH₂ | 812.59 |
| (8) | Ac-Ser-Met-Gly-Ala-Leu-Ala-NH₂ | 589.34 |
| (32) | Ac-Trp-Ala-Ala-Pro-Phe-Leu-Arg-Ala-NH₂ | 971.65 |

### EXAMPLE 6

### Muscle contraction inhibition experiment

### 6.1 Experimental animals

10 week old female SD rats weighing 220 g - 260 g.

### 6.2 Experimental instruments

HW-400S constant temperature smooth muscle trough, BL-420E biological function experimental system, tension transducer, and other instruments as conventional experimental instruments.

### 6.3 Experimental reagents

### acetylcholine chloride (Shanghai Yuanye), chloral hydrate (Shanghai Macklin).

### 6.4 Samples to be tested

Acetyl octapeptide-3(Ac-Glu-Glu-Met-Gln-Arg-Arg-Ala-Asp-NH₂), at a test concentration of 25 ppm, 50 ppm, 100 ppm, and 200 ppm, respectively;
Acetyl hexapeptide-30(Ac-Glu-Asp-Tyr-Tyr-Arg-Leu-NH₂), at a test concentration of 25 ppm, 50 ppm, 100 ppm, and 200 ppm, respectively;
the peptide (4), at a test concentration of 25 ppm, 50 ppm, 100 ppm, and 200 ppm, respectively;
the peptide (8), at a test concentration of 25 ppm, 50 ppm, 100 ppm, and 200 ppm, respectively;
the peptide (32), at a test concentration of 100 ppm, 200 ppm, 400 ppm, and 800 ppm, respectively.

### 6.5 Experimental protocol

The experimental rats were fasted except for water for 12 hours before the experiment. 10% chloral hydrate was injected intraperitoneally into the rats at a dosage of 0.1 mL/25 g, and after the rats were anesthetized, the abdominal cavity was cut open to expose the enlarged cecum. A segment of ileum was taken out from the ileocecal region and washed clean in a tyrode's solution pre-bubbled with oxygen for 10 min, and several 4 cm long segments of ileum were cut and incubated in a tyrode's solution at 4°C for subsequent use; and both ends of the ileum were tied partially and knotted with cotton threads to form a loop.

The HW-400S constant temperature smooth muscle trough and the BL-420E biological function experimental system were turned on, the tension transducer was connected to channel 1, the incubation temperature was adjusted to 37°C, the instruments were preheated for 0.5 hours, and oxygen was bubbled for more than 10 min. One end of the ileum segment is fixed to the intake bracket port with a cotton thread, and the other end thereof is connected to the tension transducer. The position and height of the transducer were appropriately adjusted to place the excised ileum segment in the center of the smooth muscle trough and then record of ileum activity was started.

After the muscle contraction was stabilized, acetylcholine with a final concentration of 0.1-1 µg/mL was added until a high and stable waveform appears in the muscle activity, and the action lasted for 5 minutes as the initial control. After balancing for 5 minutes, the samples to be tested were added respectively. Each time the test drug was replaced, the ileum segment was replaced with a new one, and relevant data was recorded by the instruments. The inhibition rate of smooth muscle contraction in ileum was calculated according to the following formula: Inhibition rate of smooth muscle contraction in ileum = (contraction force after acetylcholine administration - contraction force after test sample administration)/contraction force after acetylcholine administration x 100%

The experimental data was represented by mean standard deviation (Mean ± SD).

### 6.6 Experimental results

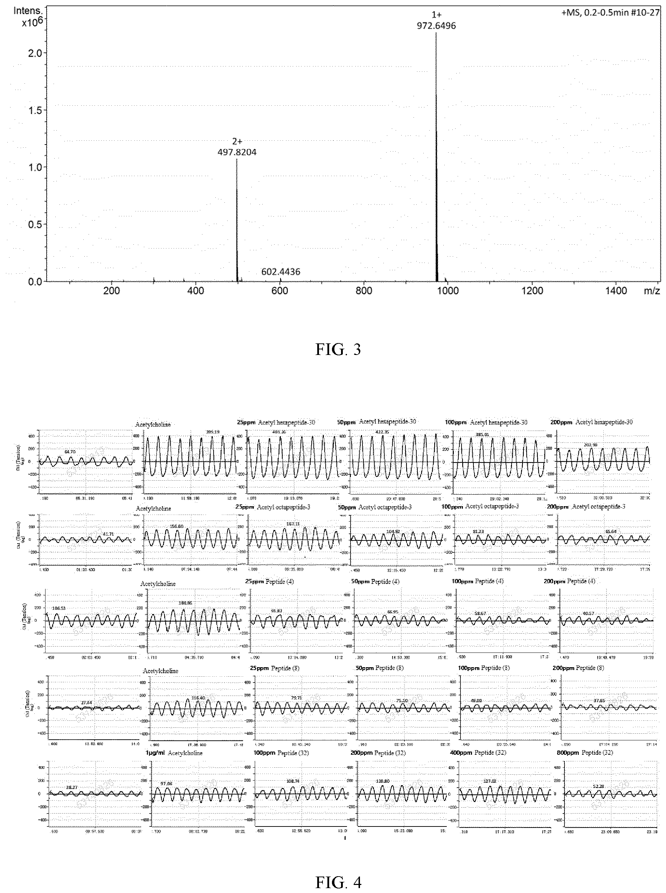

The results of the inhibition rate of smooth muscle contraction in ileum of the test samples are shown in Table 4 below and FIG. 4.

**Table 4 Inhibition rate (%) of smooth muscle contraction in ileum of test samples**

| Test sample | Concentration | | | |
|---|---|---|---|---|
| | 25 ppm | 50 ppm | 100 ppm | 200 ppm |
| Acetyl hexapeptide-30 | 0.00±2.45 | 0.47±2.09 | 2.73±0.66 | 48.09±1.35 |
| Acetyl octapeptide-3 | 10.2±1.23 | 32.34±2.26 | 47.49±1.47 | 58.67±0.41 |
| Peptide (4) | 49.78±0.35 | 62.40±1.63 | 66.72±1.93 | 76.88±0.83 |
| Peptide (8) | 31.99±3.12 | 34.35±2.30 | 56.42±1.97 | 67.96±1.05 |

The results showed that after administration of acetylcholine, a high-level signal peak could be generated. However, after administration of acetyl hexapeptide-30 at 200 ppm, this peak was significantly weakened and an inhibitory effect was exhibited with an inhibition rate of 48.09% at 200 ppm. The high-level signal peak generated by acetylcholine was inhibited after administration of acetyl octapeptide-3 at 50 ppm, with inhibition rates of 32.34% at 50 ppm, 47.49% at 100 ppm, and 58.67% at 200 ppm. However, the high-level signal peak generated by acetylcholine was significantly inhibited after administration of the peptide (4) and peptide (8) simply at 25 ppm, and the inhibitory effect was increased with the increase of concentrations. The peptide (4) exhibited the inhibitory effect at 25 ppm with an inhibition rate of 49.78%, and the inhibition rate was 62.40% at 50 ppm, 66.72% at 100 ppm, and up to 76.88% at the concentration of 200 ppm. The peptide (8) exhibited an inhibition rate of 31.99% at 25 ppm, 34.35% at 50 ppm, 56.42% at 100 ppm, and up to 67.96% at the concentration of 200 ppm. Administration of the peptide (32) at 800 ppm resulted in an inhibitory effect on muscle contraction, with an inhibition rate of 44.41%.

As can be known from the above description, the peptide of the present application has a significant inhibitory effect on muscle contraction, and the effect of the peptide of the present application is stronger than that of the polypeptides in the prior art; and the effective inhibition rate is as follows: peptide (4)>peptide (8)>acetyl octapeptide-3>acetyl hexapeptide-30.

Facial wrinkles such as wrinkles of canthus and nasolabial folds can be effectively improved by inhibiting the contraction of facial expression muscles. Acetylcholine, as a neurotransmitter, can directly activate acetylcholine receptors on smooth muscles, thus triggering a signal cascade reaction to cause the contraction of smooth muscles. By inhibiting the release of acetylcholine from cells or preventing acetylcholine from binding to its receptors, muscle contraction can be suppressed, thereby treating or caring for conditions, disorders or diseases caused by muscle contraction, treating, preventing or repairing skin aging or photoaging, and reducing, preventing or treating facial wrinkles. The peptide of the present application interferes with the interaction between Synaptophysin and VAMP2 to affect the formation of SNARE complexes from VAMP2, SNAP25, and Syntaxin, thereby inhibiting the release of acetylcholine and thus inhibiting muscle contraction. By detecting the contraction signal of ileal smooth muscles and comparing the inhibition rates of ileal smooth muscle contraction in various samples, this experiment further elucidated the technical effect of the peptide in the present application on inhibiting muscle contraction.

### EXAMPLE 7

### Preparation of an emulsion containing the peptide (4)

| Ingredients | by weight % |
|---|---|
| A cetearyl alcohol (and) cetearyl glucoside | 5.0 |
| Jojoba oil | 5.0 |
| Mineral oil | 5.0 |
| Isopropyl palmitate | 7.0 |
| B Glycerin | 5.0 |
| Allantoin | 0.1 |
| Polyacrylamide (and) C13-14 isoparaffin (and) laureth-7 | 0.3 |
| Water | Balance to |
| | 100 |
| C Preservative | 0.5 |
| D Perfume | 0.5 |
| Peptide (4) | 0.01 |

Method of formulation: the peptide (4) was formulated into a 0.02 mg/mL aqueous solution; cetearyl alcohol (and) cetearyl glucoside, jojoba oil, mineral oil, and isopropyl palmitate were heated to 85°C, and stirred evenly to afford phase A; glycerin, allantoin, polyacrylamide (and) C13-14 isoparaffin (and) laureth-7 were dissolved in water, and heated to 85°C to afford phase B; phase A was quickly added into phase B, homogenized at a constant temperature for 3-5 min and cooled; when the above mixture was cooled to below 60°C, a preservative was added and stirred evenly; when cooled to below 45°C, the polypeptide solution and perfume were added to afford an emulsion.

### EXAMPLE 8

### Preparation of an essence containing the peptide (8)

| Ingredients | by weight % |
|---|---|
| Peptide (8) | 0.02 |
| Sodium hyaluronate | 0.2 |
| Aloe vera gel | 5.0 |
| Glycerin | 5.0 |
| Vitamin C | 1.0 |
| 1,2-Octanediol | 0.25 |
| 1,2-Hexanediol | 0.25 |
| Water | Balance to 100 |
| Triethanolamine | Appropriate amount |

Method of formulation: a prescribed amount of sodium hyaluronate was added to water and stirred to mix evenly, then the mixture was heated to 80-85°C, and stirred to disperse evenly while keeping the temperature; the temperature was reduced to below 40°C, then glycerin, aloe vera gel, the peptide (8), vitamin C, 1,2-octanediol, and 1,2-hexanediol were added and stirred evenly; the pH of the solution was adjusted to around 5.5 using triethanolamine solution to afford the essence.

Although preferred embodiments of the present application have been described, those skilled in the art can make additional changes and modifications to these embodiments once they have knowledge of the basic creative concept. Therefore, the attached claims are intended to be interpreted as including the preferred embodiments and all the changes and modifications falling within the scope of the embodiments of the present application.

Finally, it should be noted that relational terms used herein such as first and second are only used to distinguish one entity or operation from another, and do not necessarily require or imply any actual relationship or order between these entities or operations. Moreover, the terms "include", "comprise", or any other variants thereof are intended to encompass non-exclusive inclusion, such that a process, method, item, or terminal device that includes a series of elements not only includes those elements, but also includes other elements not explicitly listed, or also includes elements inherent to such process, method, item, or terminal device. Without further limitations, the element defined by the statement "including one..." does not exclude the existence of other identical elements in the process, method, item, or terminal device that includes the element in question.

A polypeptide and a cosmetic composition or pharmaceutical composition and use thereof provided according to the present application are detailed in the above description. The principle and implementation of the present application are illustrated by applying specific examples herein. The description of the above embodiments is only used to help understand the method and its core idea of the present application. In addition, for those of ordinary skill in the art, changes may be made to the specific implementations and application range based on the idea of the present application. In conclusion, the contents of this specification should not be construed as a limitation to the present application.

## Claims

1. A peptide represented by Formula (I), or a stereoisomer thereof, or a mixture of the stereoisomers thereof, or a cosmetically acceptable salt thereof, or a pharmaceutically acceptable salt thereof, **characterized in that**
R₁-X₁-X₂-X₃-X₄-X₅-X₆-X₇-X₈-R₂ (I)
in Formula (I),
X₁ is selected from -Val-, -Ser-, -Trp-, -Met-, -Leu-, -Glu-, -Ala-, -Pro-, -Tyr- or -Thr-;
X₂ is selected from -Lys-, -Arg-, -His-, -Met-, -Ala-, -Leu-, -Asn-, -Trp-, -Thr-, -Pro-, -Gly-, - Phe-, -Asp-, -Ser- or -Cys-;
X₃ is selected from -Val-, -Gly-, -Leu-, -Pro-, -Met-, -Ala-, -Gln-, -Asp-, -Phe-, -Ser-, -Cys- or - Lys-;
X₄ is selected from -Gly-, -Leu-, -Pro-, -Ala-, -Phe-, -Val-, -Trp-, -Asn- or -Glu-;
X₅ is selected from -Phe-, -Trp-, -Tyr- or a bond;
X₆ is selected from -Gln-, -Leu-, -Ala-, -Val-, -Phe-, -Arg-, -Tyr-, -Gly- or -Asn-;
X₇ is selected from -Lys-, -Arg-, -His- or a bond;
X₈ is selected from -Trp-, -Ala-, -Asp-, -Lys-, -Gly-, -Tyr-, -Met- or -Arg-;
R₁ is selected from H or R₃-CO-, wherein R₃ is selected from substituted or unsubstituted alkyl, substituted or unsubstituted alkenyl;
R₂ is selected from -NR₄R₅ or -OR₄, wherein each R₄ and R₅ are independently selected from H, substituted or unsubstituted alkyl, substituted or unsubstituted alkenyl;
the alkyl refers to a saturated aliphatic linear or branched chain alkyl having 1-24 carbon atoms (optionally having 1-16 carbon atoms; optionally having 1-14 carbon atoms; optionally having 1-12 carbon atoms; or optionally having 1, 2, 3, 4, 5, or 6 carbon atoms); optionally selected from methyl, ethyl, isopropyl, isobutyl, *tert*-butyl, pentyl, hexyl, heptyl, octyl, decyl, dodecyl, tetradecyl, hexadecyl, octadecyl, 2-ethylhexyl, 2-methylbutyl, or 5-methylhexyl;
the alkenyl refers to a linear or branched chain alkenyl having 2-24 carbon atoms (optionally having 2-16 carbon atoms; optionally having 2-14 carbon atoms; optionally having 2-12 carbon atoms; or optionally having 2, 3, 4, 5, or 6 carbon atoms); the alkenyl has one or more carbon-carbon double bonds, optionally has 1, 2, or 3 conjugated or non-conjugated carbon-carbon double bonds; the alkenyl is bonded to the rest portions of a molecule through a single bond; and is optionally selected from vinyl, oleyl, or linoleyl;
optionally, the substituent in the "substituted alkyl" or "substituted alkenyl" is selected from C₁-C₄ alkyl; hydroxyl; C₁-C₄ alkoxy; amino; C₁-C₄ aminoalkyl; C₁-C₄ carbonyloxy; C₁-C₄ oxycarbonyl; halogen (such as fluorine, chlorine, bromine, and iodine); cyano; nitro; azide; C₁-C₄ alkylsulfonyl; thiol; C₁-C₄ alkylthio; C₆-C₃₀ aryloxy such as phenoxy; -NR_{b}(C=NR_{b})NR_{b}R_{c}, wherein R_{b} and R_{c} are independently selected from H, C₁-C₄ alkyl, C₂-C₄ alkenyl, C₂-C₄ alkynyl, C₃-C₁₀ cycloalkyl, C₆-C₁₈ aryl, C₇-C₁₇ aralkyl, a heterocyclic group having 3 to 10 members, or an amino protecting group.

2. The peptide represented by Formula (I) according to claim 1, or a stereoisomer thereof, or a mixture of the stereoisomers thereof, or a cosmetically acceptable salt thereof, or a pharmaceutically acceptable salt thereof, **characterized in that** R₁ is selected from H, acetyl, *tert*-butyryl, hexanoyl, 2-methylhexanoyl, capryloyl, decanoyl, lauroyl, myristoyl, palmitoyl, stearoyl, oleoyl or linoleoyl;
wherein, R₁ is selected from H, acetyl, lauroyl, myristoyl or palmitoyl;
optionally, R₁ is H or acetyl.

3. The peptide represented by Formula (I) according to claim 1, or a stereoisomer thereof, or a mixture of the stereoisomers thereof, or a cosmetically acceptable salt thereof, or a pharmaceutically acceptable salt thereof, **characterized in that** R₄ and R₅ are independently selected from H, methyl, ethyl, hexyl, dodecyl or hexadecyl;
wherein, R₄ is H and R₅ is selected from H, methyl, ethyl, hexyl, dodecyl or hexadecyl;
optionally, R₂ is -OH or -NH₂.

4. The peptide represented by Formula (I) according to claim 1, or a stereoisomer thereof, or a mixture of the stereoisomers thereof, or a cosmetically acceptable salt thereof, or a pharmaceutically acceptable salt thereof, **characterized in that** X₅ is a bond, and X₇ is a bond.

5. The peptide represented by Formula (I) according to claim 1, or a stereoisomer thereof, or a mixture of the stereoisomers thereof, or a cosmetically acceptable salt thereof, or a pharmaceutically acceptable salt thereof, **characterized in that** the peptide is selected from the following peptides (1)-(48):
(1) H-Val-Lys-Val-Leu-Gln-Trp-OH;
(2) H-Val-Lys-Val-Leu-Gln-Trp-NH₂;
(3) Ac-Val-Lys-Val-Leu-Gln-Trp-OH;
(4) Ac-Val-Lys-Val-Leu-Gln-Trp-NH₂;
(5) H-Ser-Met-Gly-Ala-Leu-Ala-OH;
(6) H-Ser-Met-Gly-Ala-Leu-Ala-NH₂;
(7) Ac-Ser-Met-Gly-Ala-Leu-Ala-OH;
(8) Ac-Ser-Met-Gly-Ala-Leu-Ala-NH₂;
(9) Ac-Val-His-Pro-Leu-Gln-Trp-OH;
(10) Ac-Val-His-Pro-Leu-Gln-Trp-NH₂;
(11) Ac-Ser-Arg-Leu-Pro-Leu-Trp-OH;
(12) Ac-Ser-Arg-Leu-Pro-Leu-Trp-NH₂;
(13) Ac-Trp-Lys-Val-Pro-Gln-Ala-OH;
(14) Ac-Trp-Lys-Val-Pro-Gln-Ala-NH₂;
(15) Ac-Val-Arg-Leu-Gly-Phe-Gln-His-Trp-OH;
(16) Ac-Val-Arg-Leu-Gly-Phe-Gln-His-Trp-NH₂;
(17) Ac-Val-His-Pro-Leu-Phe-Gln-His-Trp-OH;
(18) Ac-Val-His-Pro-Leu-Phe-Gln-His-Trp-NH₂;
(19) Ac-Val-Ala-Val-Leu-Phe-Gln-Arg-Trp-OH;
(20) Ac-Val-Ala-Val-Leu-Phe-Gln-Arg-Trp-NH₂;
(21) Ac-Val-Met-Gly-Pro-Trp-Leu-Lys-Ala-OH;
(22) Ac-Val-Met-Gly-Pro-Trp-Leu-Lys-Ala-NH₂;
(23) Ac-Val-Met-Ala-Pro-Tyr-Leu-Lys-Ala-OH;
(24) Ac-Val-Met-Ala-Pro-Tyr-Leu-Lys-Ala-NH₂;
(25) Ac-Ser-Ala-Val-Pro-Trp-Leu-Arg-Trp-OH;
(26) Ac-Ser-Ala-Val-Pro-Trp-Leu-Arg-Trp-NH₂;
(27) Ac-Ser-Met-Ala-Gly-Trp-Gln-His-Ala-OH;
(28) Ac-Ser-Met-Ala-Gly-Trp-Gln-His-Ala-NH₂;
(29) H-Trp-Ala-Ala-Pro-Phe-Leu-Arg-Ala-OH;
(30) H-Trp-Ala-Ala-Pro-Phe-Leu-Arg-Ala-NH₂;
(31) Ac-Trp-Ala-Ala-Pro-Phe-Leu-Arg-Ala-OH;
(32) Ac-Trp-Ala-Ala-Pro-Phe-Leu-Arg-Ala-NH₂;
(33) H-Trp-Lys-Ala-Leu-Phe-Gln-Arg-Trp-OH;
(34) H-Trp-Lys-Gly-Pro-Tyr-Gln-Met-Trp-OH;
(35) Ac-Met-Leu-Leu-Leu-Ala-Asp-NH₂;
(36) Ac-Val-Asn-Gln-Leu-Val-Ala-NH₂;
(37) Ac-Met-Leu-Asp-Phe-Leu-Ala-NH₂;
(38) Ac-Leu-Trp-Phe-Val-Phe-Lys-NH₂;
(39) Ac-Glu-Thr-Gly-Trp-Ala-Ala-NH₂;
(40) Ac-Ala-Pro-Phe-Leu-Arg-Ala-NH₂;
(41) Ac-Pro-Gly-Asp-Ala-Tyr-Gly-NH₂;
(42) Ac-Tyr-Gly-Asp-Ala-Gly-Tyr-NH₂;
(43) Ac-Ser-Phe-Ser-Asn-Gln-Met-NH₂;
(44) Ac-Val-Asp-Cys-Ala-Asn-Lys-NH₂;
(45) Ac-Val-Phe-Ala-Phe-Leu-Tyr-NH₂;
(46) Ac-Ser-Ser-Ala-Trp-Ala-Lys-NH₂;
(47) Ac-Thr-Cys-Lys-Glu-Leu-Arg-NH₂;
(48) Ac-Leu-Asn-Leu-Val-Leu-Trp-NH₂.

6. The peptide according to claim 5, or a stereoisomer thereof, or a mixture of the stereoisomers thereof, or a cosmetically acceptable salt thereof, or a pharmaceutically acceptable salt thereof, **characterized in that** the peptide is selected from Ac-Val-Lys-Val-Leu-Gln-Trp-NH₂, Ac-Ser-Met-Gly-Ala-Leu-Ala-NH₂, or Ac-Trp-Ala-Ala-Pro-Phe-Leu-Arg-Ala-NH₂.

7. The peptide represented by Formula (I) according to claim 1, or a stereoisomer thereof, or a mixture of the stereoisomers thereof, or a cosmetically acceptable salt thereof, or a pharmaceutically acceptable salt thereof, **characterized in that**
the cosmetically acceptable salt or pharmaceutically acceptable salt includes a metal salt of the peptide represented by Formula (I), wherein the metal includes lithium, sodium, potassium, calcium, magnesium, manganese, copper, zinc or aluminum;
optionally, the cosmetically acceptable salt or pharmaceutically acceptable salt includes a salt formed from the peptide represented by Formula (I) and an organic alkali, wherein the organic alkali includes ethylamine, diethylamine, ethylenediamine, ethanolamine, diethanolamine, arginine, lysine, histidine or piperazine;
optionally, the cosmetically acceptable salt or pharmaceutically acceptable salt includes a salt formed from the peptide represented by Formula (I) and an inorganic acid or an organic acid, wherein the organic acid includes acetic acid, citric acid, lactic acid, malonic acid, maleic acid, tartaric acid, fumaric acid, benzoic acid, aspartic acid, glutamic acid, succinic acid, oleic acid, trifluoroacetic acid, oxalic acid, pamoic acid, or gluconic acid; the inorganic acid includes hydrochloric acid, sulfuric acid, boric acid or carbonic acid.

8. A cosmetic or pharmaceutical composition, **characterized in that** it includes an effective amount of the peptide represented by Formula (I) according to any of claims 1-7, or a stereoisomer thereof, or a mixture of the stereoisomers thereof, or a cosmetically acceptable salt thereof, or a pharmaceutically acceptable salt thereof, and at least one excipient and optionally a cosmetically or pharmaceutically acceptable adjuvant;
optionally, the adjuvant is selected from a collagen synthesis stimulator, an agent that modulates the PGC-1α synthesis, an agent that modulates the activity of PPARy, an agent that increases or decreases a triglyceride content in adipocytes, an agent that stimulates or delays adipocyte differentiation, a lipolytic agent or an agent that simulates lipolysis, a fat clearing agent, an adipogenic agent, an inhibitor against acetylcholine receptor aggregation, an agent that inhibits muscle contraction, an anticholinergic agent, an elastase inhibitor, a matrix metalloproteinases inhibitor, a melanin synthesis stimulator or inhibitor, a whitening or decoloring agent, a pigmentation promoter, a self-tanning agent, an antiaging agent, a NO-synthase inhibitor, a 5α-reductase inhibitor, an inhibitor against lysyl hydroxylase and/or prolyl hydroxylase, an antioxidant, a free radical scavenger and/or an anti-air pollution agent, a reactive carbonyl species scavenger, an anti-glycation agent, an antihistamine, an antiviral agent, an antiparasitic agent, an emulsifier, an emollient, an organic solvent, a liquid propellant, a skin conditioner, a humectant, a moisture retaining substance, an alpha-hydroxy acid, a beta-hydroxy acid, a moisturizer, an epidermal hydrolase, a vitamin, an amino acid, a protein, a pigment or colorant, a dye, a biopolymer, a gelling polymer, a thickener, a surfactant, a softener, an adhesive, a preservative, an anti-wrinkle agent, an agent that can reduce or treat under-eye bags, an exfoliant, a cuticle stripping agent, a keratolytic agent, an antimicrobial, an antifungal, a fungistat, a bactericide, a bacteriostat, an agent that stimulates the synthesis of dermal or epidermal macromolecules and/or inhibits or prevents the degradation thereof, an agent that stimulates elastin synthesis, an agent that stimulates decorin synthesis, an agent that stimulates laminin synthesis, an agent that stimulates defensin synthesis, an agent that stimulates chaperone protein synthesis, an agent that stimulates cAMP synthesis, a heat shock protein, an agent that stimulates the HSP70 synthesis, an agent that stimulates the synthesis of a heat shock protein, an agent that stimulates the synthesis of hyaluronic acid, an agent that stimulates fibronectin synthesis, an agent that stimulates deacetylase synthesis, an agent that stimulates the synthesis of a lipid and a stratum corneum component, ceramide, a fatty acid, an agent that inhibits collagen degradation, an agent that inhibits elastin degradation, an agent that inhibits serine protease, an agent that stimulates fibroblast proliferation, an agent that stimulates keratinocyte proliferation, an agent that stimulates adipocyte proliferation, an agent that stimulates melanocyte proliferation, an agent that stimulates keratinocyte differentiation, an agent that inhibits acetylcholinesterase, a skin relaxant, an agent that stimulates glycosaminoglycan synthesis, an anti-hyperkeratosis agent, an comedolytic agent, an antipsoriatic agent, an anti-dermatitis agent, an anti-eczema agent, a DNA repair agent, a DNA protecting agent, a stabilizer, an antipruritic agent, an agent for the treatment and/or care of sensitive skin, a hardening agent, a tightening agent, a restructuring agent, an anti-stretching agent, an adhesive, an agent that modulates sebum production, an antiperspirant, an agent that stimulates healing, an agent that assists healing, an agent that stimulates re-epithelialization, an agent that assists re-epithelialization, a cytokine growth factor, a sedative, an anti-inflammatory agent, an anesthetic agent, an agent acting on capillary circulation and/or microcirculation, an agent that stimulates angiogenesis, an agent that inhibits vascular permeability, a venotonic agent, an agent acting on cell metabolism, an agent for improving dermis-epidermis connection, an agent that induces hair growth, a hair growth inhibiting or retarding agent, a fragrance, a chelating agent, a plant extract, an essential oil, a marine extract, an agents derived from a biological fermentation process, an inorganic salt, a cell extract, a sunscreen, and an effective organic or inorganic photoprotectant against A and/or B UV rays, or mixtures thereof.

9. The cosmetic or pharmaceutical composition according to claim 8, **characterized in that** a preparation of the cosmetic or pharmaceutical composition is selected from a cream, an oil, milk, a balm, a foam, a lotion, a gel, a liniment, sera, a soap, a shampoo, a hair conditioner, a serum, an ointment, a mousse, a pomade, a powder, a bar, a pencil, a spray, an aerosol, a capsule, a tablet, a granule, a chewing gum, a solution, a suspension, an emulsion, a syrup, an elixir, a polysaccharide film, a jelly or a gelatin;
optionally, the capsule includes a soft capsule, a hard capsule, and optionally a gelatin capsule; and the tablet includes a sugar-coated tablet.

10. A cosmetically or pharmaceutically acceptable delivery system or sustained release system, **characterized in that** it comprises an effective amount of the peptide represented by Formula (I) according to any of claims 1-7, or a stereoisomer thereof, or a mixture of the stereoisomers thereof, or a cosmetically acceptable salt thereof, or a pharmaceutically acceptable salt thereof, or the cosmetic or pharmaceutical composition according to claim 8 or 9;
the cosmetically or pharmaceutically acceptable delivery system or sustained release system is selected from a liposome, an oleosome, a nonionic surfactant liposome vesicle, an ethosome, a millicapsule, a microcapsule, a nanocapsule, a nanostructured lipid carrier, a sponge, a cyclodextrin, a lipoid vesicle, a micelle, a millisphere, a microsphere, a nanosphere, a liposphere, a microemulsion, a nanoemulsion, a milliparticle, a microparticle or a nanoparticle; or optionally a liposome or microemulsion; or optionally a water-in-oil microemulsion having an internal structure of reverse micelle.

11. Use of the peptide represented by Formula (I) according to any of claims 1-7, or a stereoisomer thereof, or a mixture of the stereoisomers thereof, or a cosmetically acceptable salt thereof, or a pharmaceutically acceptable salt thereof, or the cosmetic or pharmaceutical composition according to claim 8 or 9, or the cosmetically or pharmaceutically acceptable delivery system or sustained release system according to claim 10 in the preparation of a cosmetic composition or pharmaceutical composition for treatment or care of a condition, a disorder or a disease caused by muscle contraction, wherein the disorder or disease is dystonia.

12. Use of the peptide represented by Formula (I) according to any of claims 1-7, or a stereoisomer thereof, or a mixture of the stereoisomers thereof, or a cosmetically acceptable salt thereof, or a pharmaceutically acceptable salt thereof, or the cosmetic or pharmaceutical composition according to claim 8 or 9, or the cosmetically or pharmaceutically acceptable delivery system or sustained release system according to claim 10 in the preparation of a cosmetic composition or pharmaceutical composition for treatment, prevention or repair of skin aging or photoaging, wherein the treatment, prevention or repair of skin aging or photoaging is to reduce, prevent or treat facial wrinkles.
